# FASCICULE DE BREVET EUROPEEN

(11) **EP 0 779 075 B1**
(45) Date de publication et mention de la délivrance du brevet: **31.10.2001**
(21) Numéro de dépôt: 96402710.6
(22) Date de dépôt: 12.12.1996
(51) Int. Cl.: A61K 31/565

(54) **Application du 3alpha-hydroxy-5alpha-androsta-16-ène à titre de médicaments**
Verwendung vom 3-Alpha-Hydroxy-5Alpha-Androsta-16-ene als Arzneimittel
Use of 3-alpha-hydroxy-5alpha-androsta-16-ene as pharmaceutical

(30) Priorité: 13.12.1995 FR 9514750
(43) Date de publication de la demande: 18.06.1997
(73) Titulaire: Aventis Pharma S.A., 92160 Antony (FR)
(72) Inventeur: Philibert, Daniel, 94210 La Varenne Saint Hilaire (FR)

(56) Documents cités:
- EP-A- 0 562 843
- EP-A- 0 676 202
- WO-A-86/04236
- US-A- 5 155 045
- JOURNAL OF CHEMICAL ECOLOGY, vol. 13, no. 4, 1987, pages 717-731, XP002010793 G. PRETI ET AL.: "HUMAN AXILLARY EXTRACTS"
- "THE MERCK INDEX" 1989 , MERCK & CO., INC. , RAHWAY, N.J., U.S.A. XP002010796 * page 101, No. 672: Androst-16-en-3-ol *
- MEDICAL SCIENCE RESEARCH, vol. 15, 1987, pages 1443-1444, XP002010794 T.K. KWAN ET AL.: "16-ANDROSTENES, PUTATIVE PHEROMONES IN HUMAN SEMEN"
- BIOCHEMICAL SOCIETY TRANSACTIONS, vol. 17, no. 4, 1989, pages 749-750, XP002010795 T.K. KWAN ET AL.: "ODOROUS ANDROST-16-ENES AND OTHER C19 STEROIDS IN HUMAN SEMEN"

## Description

La présente invention concerne l'utilisation du 3alpha-hydroxy-5alpha-androsta-16-ène et ses esters pour la préparation de médicaments destinés à prévenir et/ou traiter certaines formes de stérilité masculine.

Les documents US-A-5155045 et WO-A-8604236 décrivent l'utilisation d'androsténol comme médicaments pour réguler le cycle menstruel chez la femme et pour traiter les symptômes de la ménopause. EP-A-0676202 décrit des stéroides estra 1,3,5(10) triene substitués en position 3 par un groupement 2-(dialkylamino)ethoxy, ayant une activité dans le contrôle de la fertilité masculine.
L'invention a pour objet l'application du 3alpha-hydroxy-5alpha androsta-16-ène de formule (I) : 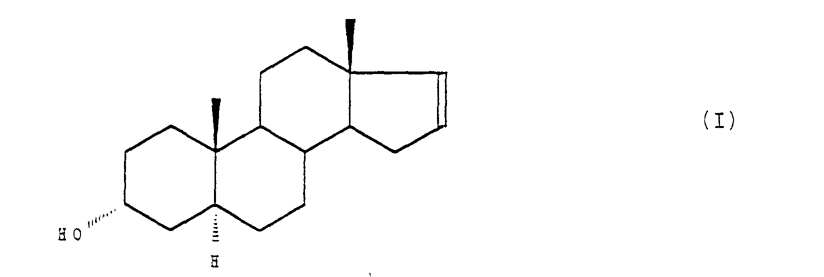 ainsi que ses esters pharmaceutiquement acceptables,pour la préparation de médicaments destinés à prévenir et/ou traiter certaines formes de stérilité masculine.

On entend par esters pharmaceutiquement acceptables les esters formés avec les acides carboxyliques aliphatiques ou aromatiques renfermant de 2 à 12 atomes de carbone. Il s'agit notamment des groupements en position 3 suivants : acétyloxy, benzoyloxy, isopropionyloxy, propionyloxy, butyryloxy, isobutyryloxy, valéryloxy, isovaléryloxy, oxalyloxy, succinyloxy, pivaloyloxy, undécanoyloxy. Il s'agit tout particulièrement du groupement acétyloxy.

La synthèse chimique du 3alpha-hydroxy-5alpha-androsta-16-ène est décrite dans Helv. Chim. Acta 27 (1944) 66 par V. Prelog et al. Les esters peuvent être préparés par les méthodes connues de l'homme du métier.

Ce produit est connu pour ses propriétés phéromonales : Gower D.B. : Quantification of odorous 16-antrostene steroids in vertebrates. In Chemical Signals in Vertebrates, 5 (Edité par D. W. Macdonald, D. Müller-Schwarze and S. E. Natynczuk). Oxford University Press, Oxford (1990).

La demanderesse a mis en évidence une nouvelle propriété des produits de formule (I) :

Les produits de l'invention présentent en effet une activité vis-à-vis de l'influx du calcium dans le spermatozoïde.

En effet, les résultats des tests montrent que ces produits agissent directement au niveau de la membrane des spermatozoïdes et stimulent l'influx de calcium dans les spermatozoïdes.

Les produits de l'invention provoquent une augmentation du calcium intracellulaire ([Ca²⁺]i) qui atteint à 10⁻⁵ M une intensité égale à celle provoquée par la progestérone (Cf. test 1).

Ils sont donc potentiellement utilisables dans le contrôle de la réaction acrosomiale et interviennent par conséquent sur le pouvoir fécondant du spermatozoïde.

Ils peuvent ainsi être utilisés dans la prévention et/ou le traitement de certaines formes de stérilité masculine caractérisées par un pouvoir fécondant insuffisant des spermatozoïdes.

Les produits de l'invention sont particulièrement avantageux car ils n'ont aucune activité hormonale ou antihormonale (cf Test 2). Il n'y a pas d'altération au niveau de la spermatogenèse et surtout de la libido.

Ces produits peuvent être utilisés chez l'homme ou chez la femme. Chez la femme, ils sont utilisés uniquement en traitement local pré ou post-coïtal.

Enfin, ces produits peuvent encore être utilisés dans le domaine vétérinaire.

La posologie utile varie en fonction de l'affection à traiter et de la voie d'administration. Elle peut varier par exemple de 10 à 1000 mg par jour chez l'homme adulte par voie orale.

Les composés de formule (I) sont utilisés par voie digestive, parentérale ou locale en particulier chez la femme, par exemple par voie percutanée, ou par voie injectable, en particulier sous-cutanée, dans le domaine vétérinaire. Ils peuvent être prescrits sous forme de comprimés simples ou dragéifiés, de gélules, de granulés, de suppositoires, de préparations injectables, d'ovules et en particulier d'ovule vaginal, de pommades, de crèmes, de gels, de microsphères, d'implants, de patchs, lesquels sont préparés selon les méthodes usuelles.

Le principe actif présente l'avantage d'être utilisable éventuellement par voie locale, notamment par voie intravaginale chez la femme.

Le ou les principes actifs peuvent être incorporés à des excipients habituellement employés dans les compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

### TESTS PHARMACOLOGIQUES

### TEST 1 : Augmentation de la concentration en calcium intracellulaire ([Ca²⁺]i) induit par la progestérone et le 3alpha-hydroxy-Salpha-androsta-16-ène (produit W) : Comparaison des effets de la progestérone à 10⁻⁵ M et du produit de l'invention à 10⁻⁵ M sur [Ca²⁺]i

### METHODE :

### Préparation des spermatozoïdes humains

Le sperme humain provient de donneurs sains. Les spermatozoides mobiles sont séparés par centrifugation sur gradient de Percoll (47,5-95 %) puis remis en suspension dans un milieu BWW (Biggers Whitten et Whittingghan) hypertonique contenant : NaCl 166 mM, KCl 5 mM, CaCl₂ 1,3 mM, KH₂PO₄ 1,2 mM, MgSO₄ 1,2 mM, glucose 5,5 mM, lactate de sodium 21 mM, pyruvate de sodium 0,25 mM, NaHCO₃ 25 mM, Hepes 20 mM et 0,8 % de HSA (Human Serum Albumin) (410 mosm/litre), pH 7,4 à température ambiante.

### Mesure du calcium intracellulaire

Les spermatozoïdes mobiles sont incubés au minimum pendant 2 heures dans le milieu capacitant BWW/HSA. Ils sont ensuite incubés (à une concentration de 5-10 x 10⁶/ml) avec du Fura2-AM (concentration finale 2 *µ*M) à 37°C pendant 45 minutes. Après lavage par centrifugation pendant 10 mn dans du BWW sans HSA, les spermatozoïdes sont remis en suspension à une concentration de 4 x 10⁶/ml. Le signal de fluorescence est mesuré à 37°C à l'aide d'un spectrofluorimètre à des longueurs d'onde d'excitation de 340 et 380 nm (PTIM 2001-Kontron) ou à 340, 360 et 380 nm (Hitachi F 2000 - B. Braun Science Tec.). L'émission de fluorescence est enregistrée à 505 nm. La progestérone ou les produits à tester, dissous dans l'éthanol absolu, sont ajoutés au milieu d'incubation à une concentration finale de 0,1 % d'éthanol. Lorsqu'un effet antagoniste de la progestérone est recherché, le produit est ajouté au milieu 2 mn avant la progestérone. A la fin de chaque dosage, 5 µM de ionomycine sont ajoutés à l'échantillon afin de mesurer le signal de fluorescence maximum ; puis les spermatozoïdes sont perméabilisés avec 0,05 % de Triton X-100 et 10 mM d'EGTA sont ajoutés (pH 9,5) afin de mesurer le signal de fluorescence minimum. Ces valeurs permettent de calculer la concentration intracellulaire en calcium ([Ca²⁺]i) selon la méthode décrite par Grunkiewicz at al (Grunkiewicz G., Poenie M. and Tsien R.Y. (1985) J. Biol. Chem. 260, 3440-3450). Les résultats des concentrations en calcium intracellulaire sont exprimés par rapport au niveau basal pris arbitrairement égal à 1.

### RESULTATS :

| Progestérone | Produit W |
|---|---|
| 6,4 | 5,69 |

Les résultats sont exprimés par rapport au niveau basal pris égal à 1. Il s'agit d'une moyenne sur 3 expériences. Les niveaux de base initiaux sont de l'ordre de 200 nm.

### CONCLUSION :

### Effet sur le calcium intracellulaire des spermatozoïdes humains

La progestérone à la concentration de 10⁻⁵ M induit une augmentation transitoire du [Ca²⁺]i suivie d'une deuxième phase où le [Ca²⁺]i est légèrement supérieur au niveau basal.

Le produit W provoque une augmentation du [Ca²⁺]i qui atteint à 10⁻⁵ M une intensité égale (88,9 % ± 36,1 %) à celle provoquée par la progestérone.

Un tel produit peut donc stimuler la réaction acrosomiale et donc être utilisé dans certaines formes de stérilité caractérisées par un pouvoir fécondant insuffisant des spermatozoïdes.

### TEST 2 : Etude de l'activité des produits de l'invention sur les récepteurs hormonaux

### METHODE :

On utilise soit le récepteur hormonal naturel de rat (récepteur androgène AR), soit le récepteur humain recombinant (récepteur progestérone PR, récepteur glucocorticoide GR, récepteur minéralocorticoide MR, récepteur estrogène ER). Les méthodes d'analyses et les méthodes de calcul sont décrites ou équivalentes à celles proposées dans la demande de brevet Européen 0269 635 A1.

### RESULTATS :

Les résultats des ARL (Affinité Relative de Liaison) sont les suivantes :

| Récepteurs | Produit W |
|---|---|
| Progestérone | < 0,1 |
| Progestérone = 100 | |
| Glucocorticoïde | < 0,1 |
| Dexaméthasone = 100 | |
| Minéralocorticoïde | < 0,1 |
| Aldostérone = 100 | |
| Estrogène | < 0,1 |
| Estradiol = 100 | |
| Androgène | < 0,1 |
| Testostérone = 100 | |

Le produit W n'a aucune affinité vis-à-vis des récepteurs hormonaux.

## Revendications

1. Application du composé de formule (I) : 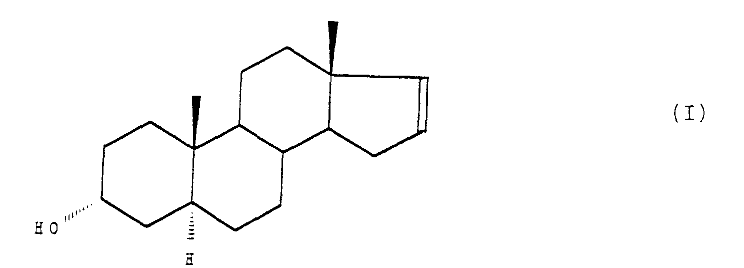 ainsi que ses esters pharmaceutiquement acceptables, pour la préparation de médicaments destinés à prévenir et/ou traiter certaines formes de stérilité masculine.

2. Application selon la revendication 1, **caractérisée en ce que** les formes de stérilité masculine sont liées au pouvoir fécondant insuffisant des spermatozoïdes.

## Claims

1. Use of the compound of formula (I): 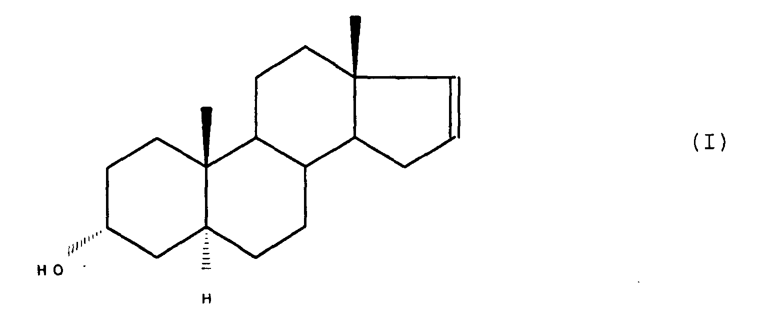 as well as its pharmaceutically acceptable esters for the preparation of medicaments intended to prevent and/or treat certain forms of male sterility.

2. Use according to claim 1, **characterized in that** the forms of male sterility are linked to the insufficient fertilizing power of the spermatozoa.

## Patentansprüche

1. Anwendung der Verbindung der Formel (I): 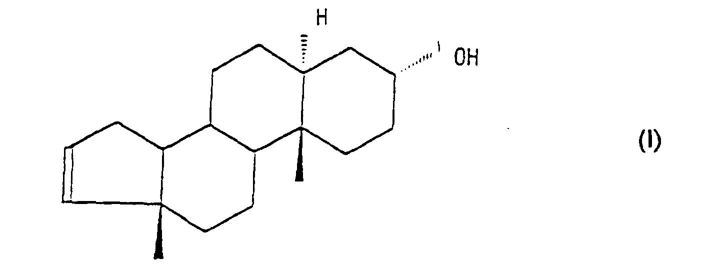 sowie ihrer pharmazeutisch verträglichen Ester für die Herstellung von Medikamenten die für die Vorbeugung und/oder Behandlung bestimmter Formen von männlicher Unfruchtbarkeit bestimmt sind.

2. Anwendung gemäß Anspruch 1, **dadurch gekennzeichnet, dass** die Formen männlicher Unfruchtbarkeit mit dem ungenügenden Befruchtungsvermögen der Spermien verbunden sind.
